# EUROPEAN PATENT SPECIFICATION

(11) **EP 2 068 729 B1**
(45) Date of publication and mention of the grant of the patent: **14.09.2011**
(21) Application number: 07840607.1
(22) Date of filing: 31.07.2007
(51) Int. Cl.: A61B 17/22, A61B 17/32

(54) **CUTTING BLADE FOR MORCELLATOR**
SCHNEIDEKLINGE FÜR EINEN MORCELLATOR
LAME COUPANT POUR SYSTÈME DE MORCELLEMENT

(30) Priority: 10.08.2006 US 502340
(43) Date of publication of application: 17.06.2009
(73) Proprietor: ETHICON, INC., Somerville, NJ 08876 (US)
(72) Inventor: NOHILLY, Martin J., Murray Hill, New Jersey 07974 (US); MIKSZA, Anthony S., Nazareth, Pennsylvania 18064 (US); NERING, Robert, Stockton, New Jersey 08559 (US)
(74) Representative: Small, Gary James
(86) International application number: PCT/US2007/074818
(87) International publication number: WO 2008/021715

(56) References cited:
- EP-A- 0 806 183
- WO-A-2006/007410
- DE-A1- 10 107 513
- DE-U1- 20 020 299

## Description

### BACKGROUND OF THE INVENTION

### Field of the Invention

The present invention relates generally to surgical devices, and more particularly to a laparoscopic morcellator having a detachable handle and various other improved features.

### Description of the Prior Art

Minimally invasive surgical procedures, such as laparoscopic procedures, have become very common. These procedures typically involve one or more small incisions that provide access to the relevant internal organ or tissue. A trocar, cannula or the like is placed into each incision, and all surgical steps are subsequently performed with instruments passed through or into the trocar(s).

Many times it is desirable to remove relatively large masses of tissue, for example a uterine fibroid, which can be difficult and time consuming given the diameter of the trocar. To this end, laparoscopic morcellators have been developed to assist in severing the tissue mass into pieces that can readily be removed through the trocar. An example of one such a morcellator is described in detail in U.S. Patent No. 6,039,748.

Known morcellators typically include a rotating tube having a sharp distal cutting edge, which rotates within an outer stationary tube. The morcellator is inserted through a cannula or trocar, or more commonly directly through the incision. A grasping instrument (i.e., tenaculum) is inserted through the inner rotating tube. Using the tenaculum, the surgeon pulls the tissue to be severed up into the tube so that the rotating edge of the inner tube severs the grasped portion of tissue. By repeating the grasping and severing procedure, the surgeon can remove the large tissue mass in increments.

Another technique surgeons have developed to improve the speed of tissue removal using a morcellator is known as "orange peeling." In orange peeling, the cylindrical blade of the morcellator is held on a plane with the outside of the organ or tissue being removed in such a way as to allow the organ or tissue to be rotated. This allows a longer strip to be removed as opposed to the "coring" technique described above, which limits the length of the strip removed to the thickness of the organ. Orange peeling requires skill of the surgeon holding the morcellator as well as skill of the assistant that is passing tissue to the morcellator with a second grasper in the cavity. The skill required is in keeping the blade at the surface of the tissue without either allowing the blade to dive in, or "core", and at the same time not leaving the surface so much that the tissue strip becomes thin or breaks. Orange peeling is better from a safety standpoint as well, as the blade remains visible at all times to the user. Thus, it would be desirable to provide a morcellator having improved feature(s) that facilitate the ability of the surgeon to use the orange peeling technique.

Another difficulty sometimes encountered with known morcellators is that during use, whether by coring or orange peelling, the amount of tissue being withdrawn can cause friction within the Inner rotating tube or to the seal system during removal. The larger the tissue sections or strips, the more exaggerated this problem becomes. It would further be desirable to provide a morcellator that lowers such withdrawal forces.

In addition to friction encountered during tissue removal, manipulation of the grasping Instrument within the rotating inner tube can interfare with the blade rotation and tends to lead to dulling of the blade with known morcellators, since the sharp edge is positioned on the inner most point on the circumference of the Inner tube. It would also be desirable to provide a morcellator that provides increased protection against such interference and blade dulling.

Finally, as indicated above, morcellators are typically inserted through a cannula, or more commonly directly through the incision When inserted directly into the incision the existing trocar must first be removed. Following morcellation, if any other procedures or tasks are to be performed within the cavity, the morcellator must be removed before any other laparoscopic instrument can be inserted through that same portal. Removal and reinsertion of trocare and laparoscopic Instruments during a given procedure is awkward and time consuming, and creates additional trauma at the site. It is further desirable to provide a morcellator that will greatly reduce the need for such exchanges.

DE 10107513 A1 discloses a hollow needle for the puncture of parenchymatous tissue, whose distal end exhibits a front cutting edge according to the preamble of claim 1.

### OBJECTS AND SUMMARY OF THE INVENTION

It is an object of the present invention to provide a morcellator cutting blade which minimizes the chances of the blade becoming dull or damaged during use as defined in independent claims 1, 2 and 3.

Features and advantages of the present invention will be apparent from the hollowing detailed description of illustrative embodiments thereof, which is to be read in connection with the accompanying drawings.

### BRIEF DESCRIPTION OF THE DRAWINGS

Figure 1 is cross-sectional view of the distal end portion of a conventional morcellator cutting blade and the end portion of a tissue grasping instrument used with the morcellator during a surgical procedure.
Figure 2 is a cross-sectional view of the distal end portion of a morcellator cutting blade as well as the end portion of a tissue grasping instrument used with a morcellator employing the cutting blade of the present invention during a surgical procedure.
Figure 3 is a cross-sectional view of the distal end portion of a cutting blade constructed in accordance with a second form of the present invention, as well as the end portion of a tissue grasping instrument used in conjunction with a morcellator employing the cutting blade of the present invention during a surgical procedure.
Figure 4 is an enlarged cross-sectional view of a portion of the cutting blade of the present invention shown in Figure 3.
Figure 5 is a cross-sectional view of the distal end portion of a cutting blade constructed in accordance with a third form of the present invention, as well as the end portion of a tissue grasping instrument used in conjunction with a morcellator employing the cutting blade of the present invention during a surgical procedure.
Figure 6 is an enlarged cross-sectional view of a portion of the cutting blade of the present invention shown in Figure 5.
Figure 7 is a cross-sectional view of the distal end portion of a cutting blade constructed in accordance with a fourth form of the present invention, as well as the end portion of a tissue grasping instrument used in conjunction with a morcellator employing the cutting blade of the present invention during a surgical procedure.
Figure 8 is an enlarged cross-sectional view of a portion of the cutting blade of the present invention shown in Figure 7.
Figure 9 is a perspective view of the distal end portion of a cutting blade for a morcellator.
Figure 10 is a side view of the distal end portion of the cutting blade shown in Figure 9.
Figure 11 is a side view of the distal end portion of a cutting blade for a morcellator.
Figure 12 is a cross-sectional view of the distal end portion of a cutting blade for a morcellator.
Figure 13 is an enlarged cross-sectional view of a portion of the cutting blade shown in Figure 12.

### DETAILED DESCRIPTION OF THE PREFERRED EMBODIMENTS

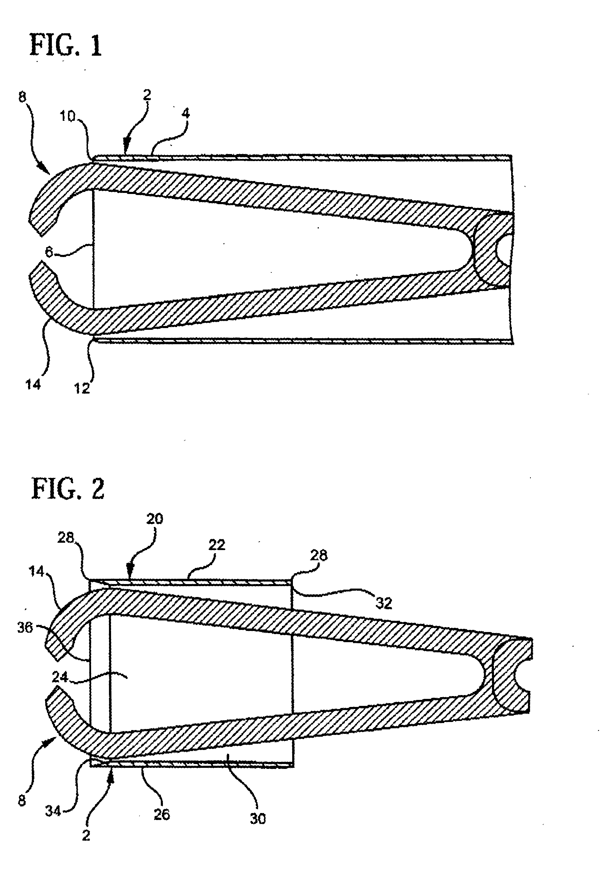

To facilitate an understanding of the present invention, reference should be made to Figure 1 of the drawings, which shows the distal end 2 of a cutting blade 4 of a conventional morcellator. The morcellator has a cylindrical cutting blade 4 that rotates within an outer fixed tube or sleeve (not shown). The cutting blade 4 defines an axial bore 6 in which is selectively received a tissue grasping tool, or tenaculum 8. The exposed cutting edge 10 of the conventional morcellator blade 4 is sharpened in the cylindrical plane of the radially inner surface 12 of the cutting blade, as can be seen in Figure 1.

The tenaculum 8 includes two expandable grasping claws or hooks 14 which are intended to grasp the tissue of an anatomical body (e.g., organ) and pull the tissue toward the rotating cutting blade 4 of the morcellator so that it may be cut into tissue "morsels". The claws 14, after grasping tissue between them, may be in an expanded or spread state, as the tenaculum 8 is pulled toward and through the axial bore 6 of the cutting blade in order to transect the tissue and remove the severed tissue morsel from the patient's body through the axial bore 6 of the cutting blade. When the tenaculum claws 14 are in this expanded state, it is possible for them to contact the sharpened cutting blade edge 10 of the morcellator. Both the tenaculum 8 and the cutting blade 4 of the morcellator are made from metal, preferably stainless steel, and this metal-to-metal contact dulls the blade during insertion and withdrawal of the tenaculum respectively into and from the morcellator. A damaged or dull blade can excessively prolong the laparoscopic surgery using the morcellator. Alternatively, the tenaculum 8 may be off center with respect to the axis of the morcellator cutting blade 4 and contacts the sharpened cutting edge 10 when the tenaculum grasps tissue and pulls it toward the morcellator.

As shown in Figure 2 of the drawings, a cutting blade 20 for a morcellator is formed as an elongated tubular member having a cylindrically-shaped sidewall 22 defining an axial bore 24 for the passage of transected tissue morsels therethrough. The sidewall 22 has an outer surface 26 residing in a first cylindrical plane 28 and an inner surface 30 disposed radially inwardly of the outer surface 26 and residing in a second cylindrical plane 32 concentrically situated within the first cylindrical plane 28. The sidewall 22 further includes a beveled or sloped surface 34 that extends from the inner surface 30 toward the outer surface 26 in a direction toward the exposed free end of the cutting blade to define a sharpened edge 36 in the first cylindrical plane 28 in which the outer surface 26 resides.

The purpose of having the sharpened edge 36 on the outside surface 26 of the cutting blade, as opposed to the inside surface 12 on conventional morcellator cutting blades, is that, when the tenaculum 8 is withdrawn through the morcellator, with its claws 14 spread to some degree, it will contact the inside surface 30 of the cutting blade 20 and not the sharpened edge 36, as can be clearly seen in Figure 2 of the drawings.

The slope of the beveled surface 34 is preferably about 15 degrees measured as the acute exterior angle A formed between the sloped surface and the first cylindrical plane 28 in which the outer surface 26 of the morcellator blade sidewall 22 generally resides. This angle is preferred as a compromise between obtaining a sharp edge on the cutting blade 20 and protecting the cutting edge 36 against inadvertent contact with the claws 14 of the tenaculum 8.

More specifically, it may not be obvious to a surgeon when viewing the surgical procedure through an endoscope that the claws 14 of the tenaculum are spread to such an extent that the tenaculum 8 will contact the cutting blade 20 if the tenaculum is withdrawn through the morcellator. The angle of the beveled surface 34 of the cutting blade 20 is chosen to be preferably about 15 degrees to protect the sharpened edge 36 against such inadvertent contact with the tenaculum 8 under such circumstances when it is not readily apparent to the surgeon that contact between the tenaculum and the sharpened edge will occur, and still provide a sharp edge for cutting. When it is clearly obvious to an astute surgeon that the tenaculum 8, when grasping tissue for transection, is open to such a degree that it will contact the rotating sharpened edge 36 of the morcellator blade 20, the surgeon will not attempt to pull the tenaculum through the morcellator- he or she will release the tissue and grasp a smaller quantity. Accordingly, the preferred 15 degree slope is chosen as a precautionary angle to protect the cutting blade 20 when it is not so obvious to the surgeon that contact between the tenaculum 8 and the sharpened blade edge 36 will occur. This angle is based on experimentation and observation and may vary widely.

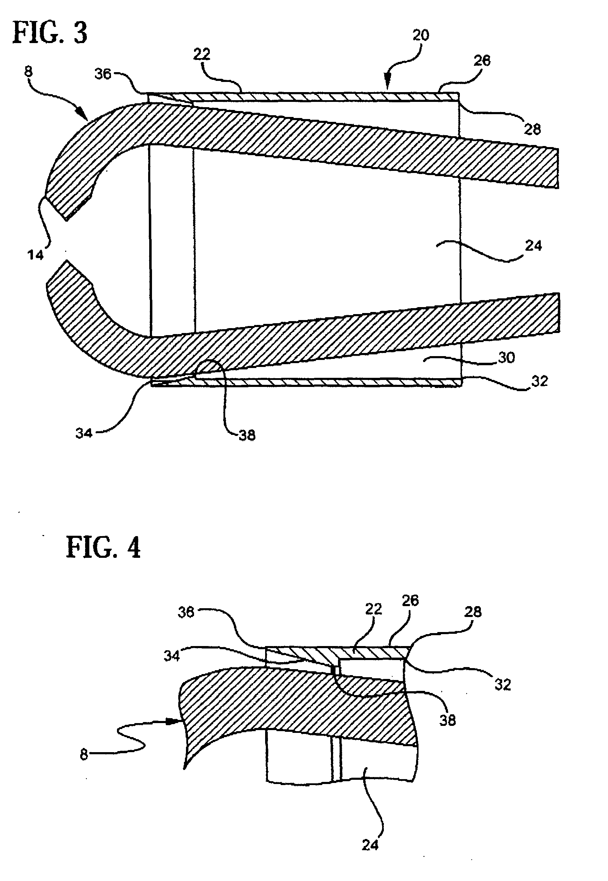

Figures 3 and 4 of the drawings illustrate A cutting blade 20 for a morcellator constructed in accordance with the present invention. Here, the beveled or sloped surface 34 of the cutting blade sidewall 22, near the distal free end of the blade 20, continues beyond the general cylindrical plane 32 of the inner surface 30 to define a shoulder 38 that projects radially into the axial bore 24 of the cutting blade. This shoulder 38 can be fabricated integrally with the cutting blade sidewall 22, or separately and mounted thereon, and can be continuous or interrupted circumferentially about the inner surface 30 of the cutting blade sidewall. The shoulder 38, when contacted by the claws 14 of the tenaculum 8, further prevents the tenaculum from engaging the sharpened blade edge 36 which, again, is preferably situated in the cylindrical plane 28 in which the outer surface 26 of the cutting blade sidewall resides, as in the embodiment shown in Figure 2 of the drawings.

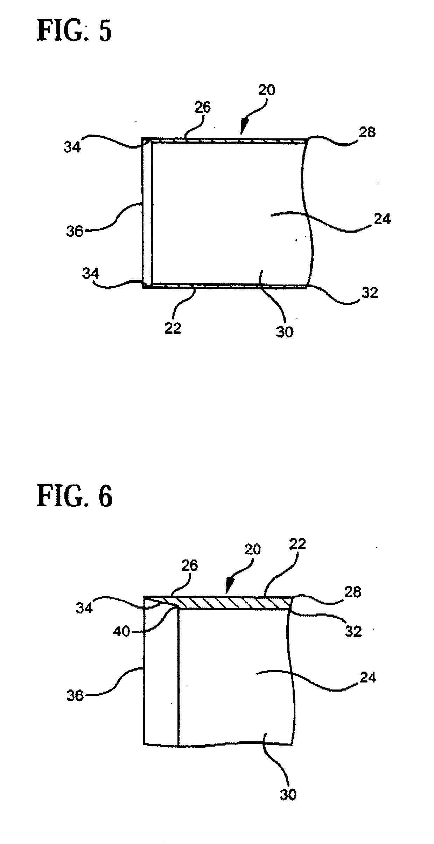

Figures 5 and 6 illustrate an alternative form of the cutting blade 20 of the present invention shown in Figures 3 and 4. The cutting blade 20 is formed with an edge 40 on the inner surface 30 of its sidewall 22 that radially extends outwardly towards the outer surface 26 of the cutting blade sidewall, where it meets the sloped surface 34. Again, as in the previous embodiments of the present invention, the sloped surface 34 preferably defines with the outer surface 26 a sharpened edge 36 which resides in the cylindrical plane 28 of the outer surface. Thus, the edge 40 defines the cutting blade 20 with a stepped inner surface that is engageable by the claws 14 of the tenaculum 8, rather than the sharpened cutting edge 36 of the blade being contacted, in order to protect the sharpened blade from dulling, becoming outwardly flared or chipping as the tenaculum 8 is withdrawn through or extended from the axial bore 24 of the cutting blade.

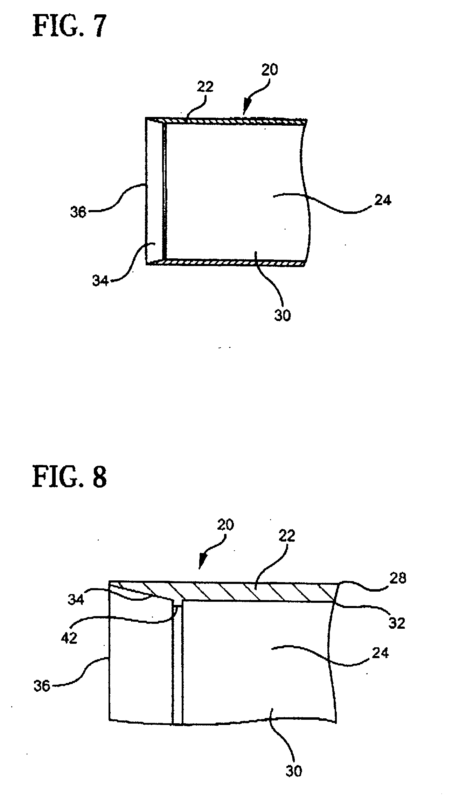

Figures 7 and 8 illustrate another form of a morcellators cutting blade 20 constructed in accordance with the present invention. On the inner surface 30 of the cutting blade sidewall 22, in proximity to where the sloped surface 34 meets the inner surface opposite the sharpened cutting edge 36, is situated a raised ring 42 or bump that extends radially into the axial bore 24 of the cutting blade. The ring 42 is again provided so that the claws 14 of the tenaculum 8 will engage the ring 42 rather than contacting the sharpened blade edge 36 and dulling or damaging the cutting edge. The raised ring 42 or bump may be integrally formed with the cutting blade sidewall 22 on the inner surface 30 thereof. Alternatively, the inner surface 30 may include a recess formed therein and circumferentially extending about the cutting blade sidewall 22, into which is captively received and seated therein a portion of the raised ring 42.

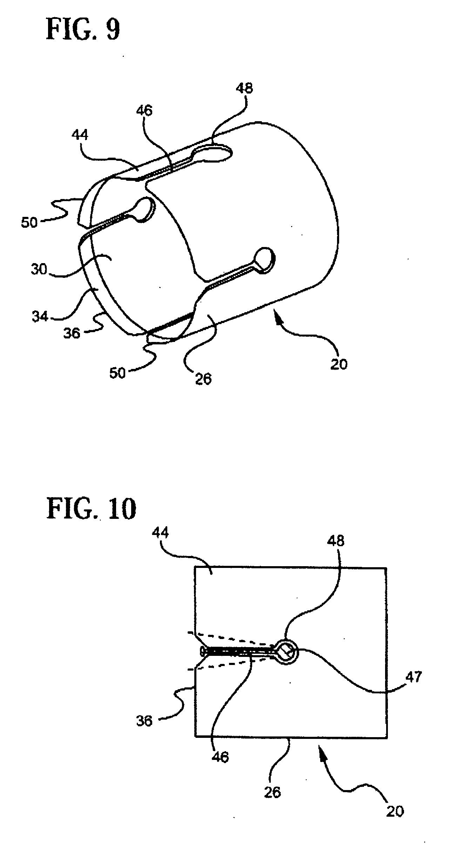

Figures 9 and 10 show another form of a cutting blade 20 for a morcellator constructed not in accordance with the present invention. In this particular embodiment, at least a portion of the cutting blade is made flexible so that it may deflect when the tenaculum 8 strikes it.

More specifically, and as shown in Figures 9 and 10, the distal end portion 44 of the cutting blade 20 is made flexible by forming slots 46 in the blade sidewall 22 extending axially along a portion of the longitudinal length thereof, which slots 46 are spaced apart periodically about the circumference of the cutting blade 20. The slots 46 extend to the sharpened cutting edge 36, and terminate inwardly of the cutting edge in respective round openings 48 formed through the thickness of the cutting blade wall 22. Preferably, as in the other embodiments previously described, a beveled or sloped surface 34 of the cutting blade sidewall 22 extends from the inner surface 30 in the direction of the distal end of the blade and meets the outer surface 26 to define a sharp cutting edge 36 situated in the cylindrical plane 28 in which the outer surface of the cutting blade sidewall resides. However, it is envisioned to be within the scope of the present invention to form the sharpened edge 36 on the inner surface 30 of the cutting blade sidewall 22, with the outer surface 26 joining the sloped surface 34 which extends toward the inner surface to form a sharpened edge 36 on the cutting blade that resides in the cylindrical plane 32 in which the inner surface 30 resides. With this particular embodiment, when the tenaculum 8 and in particular, the claws 14 thereof, engage the cutting blade 20 of the morcellator, the slotted portions 50 of the blade will give or deflect out of the way, so as not to be damaged or dulled when contacted by the tenaculum.

The slots 46 in the cutting blade sidewall 22 may be filled with an elastomeric or polymeric material 47. The slots 46 are filled so that the material 47 is flush with the inner and outer surfaces 30, 26 of the cutting blade sidewall 22. The purpose of filling the slots 46 with an elastomeric or polymeric material 47 is to prevent tissue that is being cut from getting caught in the slots 46 and creating friction or damage to the cutting blade 20, or preventing the cutting blade from rotating within the outer sleeve of the morcellator.

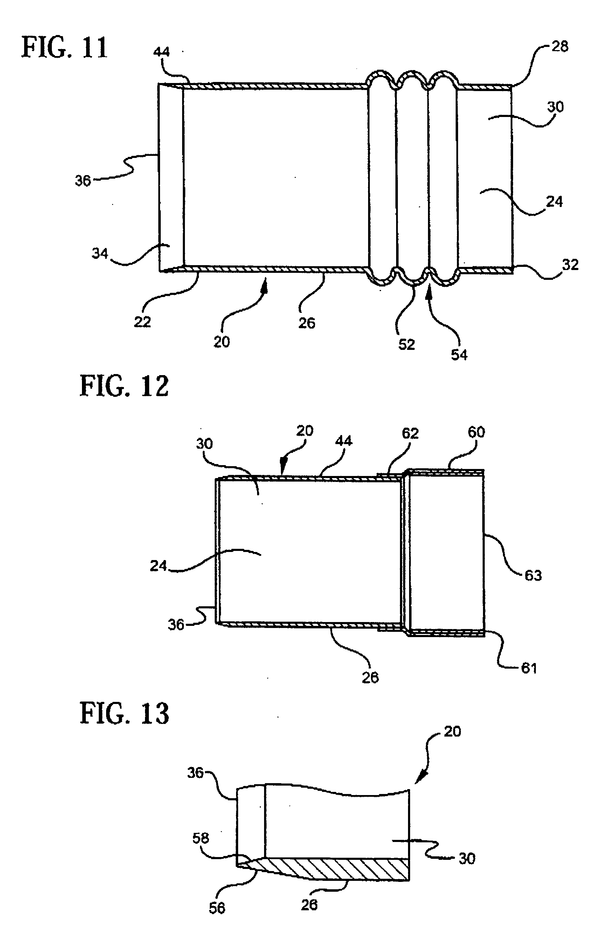

Figure 11 illustrates yet another embodiment of a morcellator cutting blade 20 formed not in accordance with the present invention. Here, the cutting blade 20 is made flexible so that it is self-centering on the tenaculum 8.

More specifically, a portion of the longitudinal length of the cutting blade 20 is formed with undulations 52 in its sidewall 22 to define a flexible joint 54 thereat. The flexible joint 54 allows the distal end of the blade 20 to float and be self-centering on the tenaculum 8. Again, preferably the cutting blade sidewall 22 has a sloped or beveled surface 34 that extends from the inner surface 30 in the distal end direction to the outer surface 26 to form with the outer surface a sharpened cutting edge 36 situated in the cylindrical plane 28 in which the outer surface resides.

The flexible joint portion 54 of the cutting blade may be formed from a separate molded elastomeric or plastic material piece that joins the distal end portion 44 of the cutting blade, which distal end portion 44 may be formed from stainless steel or other metal. Alternatively, the flexible joint 54 may be a series of ripples or undulations 52 formed directly in the metal tubular sidewall 22 of the cutting blade. Oftentimes, the tenaculum 8 is forced to extend at an angle to the axis of the cutting blade 20 to reach anatomical tissue structures. With this embodiment, the cutting blade 20 of the present invention will flex in the direction which the tenaculum 8 extends in order to minimize the chance that the tenaculum will contact the sharpened cutting edge 36 of the blade. The flexible joint 54 a lows the cutting blade to be self-centering on the tenaculum 8 which is situated in the axial bore 24 thereof.

A morcellator cutting blade 20 may also be formed with a sharpened edge 36 disposed between the cylindrical planes 28, 32 in which the inner surface 30 and the outer surface 26 of the cutting blade reside. This particular embodiment is shown in Figures 12 and 13 of the drawings.

More particularly, the sidewall 22 of the cutting blade 20 includes a first sloped or beveled surface 56 which extends from the inner surface 30 of the sidewall 22 and is angled toward the outer surface 26 in the direction of the distal end of the cutting blade. Similarly, the sidewall 22 of the cutting blade further includes a second beveled or sloped surface 58 which extends from the outer surface 26 and is angled toward the inner surface 30 in the direction of the distal end of the cutting blade. Together, the first and second sloped surfaces 56, 58 meet to define the sharpened edge 36 of the cutting blade. The sharpened edge 36 is disposed between the cylindrical planes in which the inner surface 30 and the outer surface 26 of the cutting blade sidewall reside. Preferably, the first sloping surface 56 forms an acute exterior angle B with respect to the plane 32 in which the inner surface 30 of the cutting blade sidewall resides, which angle B is about 15 degrees. Similarly, the second sloped surface 58 forms an acute exterior angle C with respect to the plane 28 in which the outer surface 26 resides, which angle C is about 11 degrees. The first sloped surface 56 of the sidewall 22 is provided in this embodiment of the cutting blade, like the previous embodiments described herein, to protect the sharpened edge 36 from the tenaculum 8 when it is not obvious to the surgeon that the claws 14 of the tenaculum are spread apart too wide and would have contacted the sharpened edge 36 of the cutting blade had the cutting blade 20 been formed conventionally with its sharpened edge residing in the cylindrical plane 32 in which the inner surface 30 of the cutting blade sidewall resides.

The distal end of the morcellator cutting blade 20 is preferably made from surgical stainless steel, and even more preferably, a hardened and or coated steel which is easier to keep sharp. Stainless steel is preferred because it will not corrode and provides the axial bore 24 of the cutting blade 20 with a smooth, polished surface which minimizes friction between the cutting blade 20 and transected morsels being pulled through the axial bore 24 of the rotating cutting blade of the morcellator. Even more preferably, the distal end 44 of the cutting blade 20 of the present invention is formed from Nos. 304, 316, 316L, or 420, 465 grade stainless steel, although it is envisioned to be within the scope of the present invention to form the distal end of the cutting blade from other grades of surgical stainless steel and from other materials. Furthermore, the cutting blade 20 may be titanium coated on its inside surface and outside surface for extra durability and/or low friction.

It is also envisioned form the cutting blade 20 of the morcellator from different materials or from different grades of materials. As shown in Figures 12 and 13 of the drawings, the distal end portion 44 of the cutting blade may be formed from a rather expensive, surgical stainless steel, and or coated surgical stainless steel to improve the hardness properties, to maintain the sharpness and hardness of the cutting edge 36, while the remainder or adjoining portion 60 of the morcellator blade may be formed from a different material, such as a different grade surgical stainless steel or from a thermoplastic material. For example, the adjoining portion 60 of the cutting blade 20 may be formed from a surgical stainless steel of No. 301, 302, 303, 304 grade or the like, which is not as relatively expensive as the material from which the distal end portion 44 of the cutting blade is formed, as the adjoining portion 60 need not retain its hardness and does not define the sharpened edge. It is preferred, however, that the adjoining portion 60 of the cutting blade still provide a polished inner surface 30 to reduce friction between transected morsels and the rotating cutting blade as the tissue morsels are being pulled through the axial bore 24 of the morcellator cutting blade. The distal end 44 of the cutting blade may be affixed to the adjoining portion 60 by welding or brazing the two materials together to form a unitary joint. Alternatively, and as shown in Figures 12 and 13 of the drawings, the mating end of the distal end portion 44 of the cutting blade sidewall 22 opposite the sharpened edge 36 may be at least partially closely received by a sleeve 62 formed by machine rolling the corresponding mating end of the adjoining portion 60 of the cutting blade to a smaller inner diameter that approximates the outer diameter of the distal end portion 44 of the cutting blade sidewall 22. The distal end portion 44 that is closely received by the sleeve 62 of the adjoining portion 60 may be welded or adhesively secured to the sleeve.

As mentioned previously, the adjoining portion 60 of the cutting blade may be also formed from a polymeric or elastomeric material. In this case, the mating end of the stainless steel distal end portion 44 of the blade may be attached to the polymeric or elastomeric adjoining portion 60, for example by being closely received by a sleeve 62 formed on the mating end of the adjoining portion 60 of the cutting blade and adhesively secured thereto in much the same way as described previously. Polymeric and elastomeric materials which may be used to form the adjoining portion 60 of the cutting blade include, but are not limited to, PEEK (polyetheretherketone), Polycarbonate, and Nylon. Furthermore, it would be preferred if the adjoining portion 60 of the cutting blade were formed from a material which is inherently lubricious, or the inner surface 30 thereof were to include a lubricious coating, in order to minimize friction between transected tissue morsels and the inner surface 30 of the cutting blade, as the tissue morsels are being pulled by the tenaculum 8 through the axial bore 24 of the cutting blade.

Another feature of the morcellator cutting blade 20 formed as illustrated by Figures 12 and 13 of the drawings. It is known that with conventional morcellator cutting blades 4, friction between transected tissue morsels and the rotating cutting blade may cause the morsels to tear from the tenaculum 8 and become dislodged and entrapped within the axial bore 6 of the cutting blade. This occurs more frequently when long strands of transected tissue resulting from a commonly used surgical technique referred to as "orange peeling" are pulled through the morcellator. The long tissue strands twist and turn within the axial bore 6 of the rotating cutting blade as the tenaculum 8 is being pulled through the morcellator. Also, oversized transected tissue morsels which are too large to move freely within the axial bore 6 of the cutting blade 4 may create excessive friction with the inner surface 12 of the cutting blade, become dislodged from the tenaculum 8 and plug the axial bore 6, which may require the morcellator to be removed from the patient, and the obstructing tissue removed from the morcellator bore. Or, the entrapped tissue morsels must be pushed back out the distal end 2 of the morcellator cutting blade 4 where they can be regrasped by the tenaculum claws 14 and pulled through the morcellator again. Once entrapped in the axial bore 6 of the morcellator cutting blade 4, the tissue morsel may be difficult to grasp, as the tenaculum claws 14 may not be spread wide enough within the axial bore of the cutting blade to securely engage the entrapped tissue. Also, it may be necessary to remove power to the morcellator in order to prevent the cutting blade 4 from rotating, as the entrapped tissue morsel will spin within the axial bore 6 with the rotating cutting blade, making it even more difficult to extract.

The above-described problem with conventional morcellators has been addressed by the present invention.

Although illustrative embodiments of the present invention have been described herein with reference to the accompanying drawings, it is to be understood that the invention is not limited to those precise embodiments, and that various other changes and modifications may be effected therein by one skilled in the art without departing from the scope of the invention as defined by the claims.

## Claims

1. A cutting blade (20) for a surgical morcellator, the cutting blade (20) having a distal end and a sharpened cutting edge (36) situated at the distal end for transecting tissue to form tissue morsels, the cutting blade (20) being structured to minimize contact between the sharpened edge (36) and a tissue gasping instrument (2) used with the morcellator during a surgical procedure, the cutting blade (20) comprising: a generally cylindrically-shaped sidewall (22) defining an axial bore for the passage of transected tissue morsels therethrough, the sidewall (22) having an outer surface (26) residing generally in a first cylindrical plane (28) an inner surface (30) residing generally in a second cylindrical plane (32) and a sloped surface (36), the inner surface of (30) the sidewall (22) being disposed radially inwardly of the outer surface (26) of the sidewal (22) the sloped surface (34) extending transversely between the inner surface (30) and the outer surface (26) in the direction of the distal end of the cutting blade (20) and joining the outer surface (36) at an acute angle to define with the outer surface (26) the sharpened cutting edge (36) the sharpened cuttittg edge (36) reasiding in the first cylindrical plane in which the outer surface (26) of the sidewall (22) generally resides, **characterized in that** the sloped surface (34) of the cutting blade sidewall (28) extends radially inwardly beyond the second cylindrical plane (32) in which the inner surface (30) the cutting blade sidewall (22) generally resides to define a circular protrusion (30) situated radially inwardly of the inner surface (30) for selective engagement with the tissue grasping instrument (8) used with the morcellator during a surgical procedure.

2. A cutting blade (20) for a surgical morcellator, the cutting blade (20) having a distal end and a sharpened cutting edge (36) situated at the distal end for transecting tissue to form tissue morsels, the cutting blade (20) being structured to minimize contact between the sharpened edge (36) and a tissue gasping instrument (2) used with the morcellator during a surgical procedure, the cutting blade (20) comprising a generally cylindrically-shaped sidewall (22) defining an axial bore for the passage of transected tissue morsels therethrough, the sidewall (22) having an outer surface (26) residing generally in a first cylindrical plane (28) an inner surface (30) residing generally in a second cylindrical plane (32) and a sloped surface (36), the inner surface of (30) the sidewall (22) being disposed radialy inwardly of the outer surface (26) of the sidewal (22) the sloped surface (34) extending transversely between the inner surface (30) and the outer surface (26) in the direction of the distal end of the cutting blade (20) and joining the outer surface (26) at an acute angle to define with the outer surface (26) the sharpened cutting edge (36) the sharpened cutting edge (36) reasiding in the first cylindrical plane in which the outer surface (26) of the sidewall (22) generally resides, **characterized in that** the inner surface (30) of the cutting blade sidewall (22) includes a radially outwardly extending portion, the sloped surface (34) of the cutting blade sidewall (36) extending between the radially outwardly extending portion and the outer surface (26) of the cutting blade sidewall (22) the radially outwardly extending portion of the inner surface (30) defining a radial edge (40) in the cutting blade sidewall (22) for selective engagement with the tissue grasping instrument (8) used with the morcellator during a surgical procedure.

3. A cutting blade (20) for a surgical morcellator, the cutting blade (20) having a distal end and a sharpened cutting edge (36) situated at the distal end for transecting tissue to form tissue morsels, the cutting blade (20) being structured to minimize contact between the sharpened edge (36) and a tissue gasping instrument (2) used with the morcellator during a surgical procedure, the cutting blade (20) comprising: a generally cylindrically-shaped sidewall (22) defining an axial bore for the passage of transected tissue morsels therethrough, the sidewall (22) having an outer surface (26) residing generally in a first cylindrical plane (28) an inner surface (30) residing generally in a second cylindrical plane (32) and a sloped surface (36), the inner surface of (30) the sidewall (22) being disposed radially inwardly of the outer surface (26) of the sidewal (22) the sloped surface (34) extending transversely between the inner surface (30) and the outer surface (26) in the direction of the distal end of the cutting blade (20) and joining the outer surface (36) at an acute angle to define with the outer surface (26) the sharpened cutting edge (36) the sharpened cuttittg edge (36) reasiding in the first cylindrical plane in which the outer surface (26) of the sidewall (22) generally resides, **characterized in that** the cutting blade (20) further includes a ring (42) the ring (42) being situated on the inner surface (34) of the sidewall (23) and extending radially therefrom and partially into the axial bore (24) the ring (42) being provided for selective engagement with the tissue grasping instrument (8) used with the morcellator during a surgical procedure.

4. A cutting blade (20) for a surgical morcellator as defined by any preceding claim, wherein the sloped surface (34) of the cutting blade (20) forms with the second cylindrical plane (32) in which the inner surface (30) of the cutting blade sidewall (32) generally resides an exterior angle of about 15 degrees (15°).

## Patentansprüche

1. Schneidklinge (20) für einen chirurgischen Morcellator, wobei die Schneidklinge (20) ein distales Ende und eine geschärfte Schneidkante (36) am distalen Ende zum Durchschneiden von Gewebe zur Bildung von Gewebestücken aufweist, wobei die Schneidklinge (20) eine solche Struktur aufweist, dass Kontakt zwischen der geschärften Kante (36) und einem mit dem Morcellator während eines chirurgischen Eingriffs verwendeten Gewebegreifinstrument (2) minimiert wird, wobei die Schneidklinge (20) Folgendes umfasst: eine allgemein zylinderförmige Seitenwand (22), die eine axiale Bohrung zur Durchführung durchgeschnittener Gewebestücke durch sie hindurch definiert, wobei die Seitenwand (22) eine Außenseite (26), die im Allgemeinen in einer ersten zylinderförmigen Ebene (28) liegt, eine Innenseite (30), die im Allgemeinen in einer zweiten zylinderförmigen Ebene (32) liegt, und eine geneigte Fläche (34) aufweist, wobei die Innenseite (30) der Seitenwand (22) von der Außenseite (26) der Seitenwand (22) radial einwärts angeordnet ist, wobei sich die geneigte Fläche (34) quer zwischen der Innenseite (36) und er Außenseite (26) in Richtung des distalen Endes der Schneidklinge (20) erstreckt und an die Außenseite (26) in einem spitzen Winkel anschließt, um mit der Außenseite (26) die geschärfte Schneidkante (36) zu definieren, wobei die geschärfte Schneidkante (36) in der ersten zylinderförmigen Ebene liegt, in der die Außenseite (26) der Seitenwand (22) im Allgemeinen liegt, **dadurch gekennzeichnet, dass** sich die geneigte Fläche (34) der Schneidklingen-Seitenwand (22) radial nach innen über die zweite zylinderförmigen Ebene (32), in der die Innenseite (30) der Schneidklingen-Seitenwand (22) im Allgemeinen liegt, hinaus erstreckt, um einen kreisförmigen Vorsprung (39) zu definieren, der radial einwärts von der Innenseite (30) zum selektiven Eingriff mit dem Gewebegreifelement (8), das während eines chirurgischen Eingriffs zusammen mit dem Morcellator verwendet wird, angeordnet ist.

2. Schneidklinge (20) für einen chirurgischen Morcellator, wobei die Schneidklinge (20) ein distales Ende und eine geschärfte Schneidkante (36) am distalen Ende zum Durchschneiden von Gewebe zur Bildung von Gewebestücken aufweist, wobei die Schneidklinge (20) eine solche Struktur aufweist, dass Kontakt zwischen der geschärften Kante (36) und einem mit dem Morcellator während eines chirurgischen Eingriffs verwendeten Gewebegreifinstrument (2) minimiert wird, wobei die Schneidklinge (20) Folgendes umfasst: eine allgemein zylinderförmige Seitenwand (22), die eine axiale Bohrung zur Durchführung durchgeschnittener Gewebestücke durch sie hindurch definiert, wobei die Seitenwand (22) eine Außenseite (26), die im Allgemeinen in einer ersten zylinderförmigen Ebene (28) liegt, eine Innenseite (30), die im Allgemeinen in einer zweiten zylinderförmigen Ebene (32) liegt, und eine geneigte Fläche (34) aufweist, wobei die Innenseite (30) der Seitenwand (22) von der Außenseite (26) der Seitenwand (22) radial einwärts angeordnet ist, wobei sich die geneigte Fläche (34) quer zwischen der Innenseite (36) und er Außenseite (26) in Richtung des distalen Endes der Schneidklinge (20) erstreckt und an die Außenseite (26) in einem spitzen Winkel anschließt, um mit der Außenseite (26) die geschärfte Schneidkante (36) zu definieren, wobei die geschärfte Schneidkante (36) in der ersten zylinderförmigen Ebene liegt, in der die Außenseite (26) der Seitenwand (22) im Allgemeinen liegt, **dadurch gekennzeichnet, dass** die Innenseite (30) der Schneidklingen-Seitenwand (22) einen sich radial nach außen erstreckenden Abschnitt aufweist, wobei sich die geneigte Fläche (34) der Schneidklingen-Seitenwand (22) zwischen dem sich radial nach außen erstreckenden Abschnitt und der Außenseite (26) der Schneidklingen-Seitenwand (22) erstreckt, wobei der sich radial nach außen erstreckende Abschnitt der Innenseite (30) eine radiale Kante (40) in der Schneidklingen-Seitenwand (22) zum selektiven Eingriff mit dem Gewebegreifinstrument (8), das während eines chirurgischen Eingriffs zusammen mit dem Morcellator verwendet wird, definiert.

3. Schneidklinge (20) für einen chirurgischen Morcellator, wobei die Schneidklinge (20) ein distales Ende und eine geschärfte Schneidkante (36) am distalen Ende zum Durchschneiden von Gewebe zur Bildung von Gewebestücken aufweist, wobei die Schneidklinge (20) eine solche Struktur aufweist, dass Kontakt zwischen der geschärften Kante (36) und einem mit dem Morcellator während eines chirurgischen Eingriffs verwendeten Gewebegreifinstrument (2) minimiert wird, wobei die Schneidklinge (20) Folgendes umfasst: eine allgemein zylinderförmige Seitenwand (22), die eine axiale Bohrung zur Durchführung durchgeschnittener Gewebestücke durch sie hindurch definiert, wobei die Seitenwand (22) eine Außenseite (26), die im Allgemeinen in einer ersten zylinderförmigen Ebene (28) liegt, eine Innenseite (30), die im Allgemeinen in einer zweiten zylinderförmigen Ebene (32) liegt, und eine geneigte Fläche (34) aufweist, wobei die Innenseite (30) der Seitenwand (22) von der Außenseite (26) der Seitenwand (22) radial einwärts angeordnet ist, wobei sich die geneigte Fläche (34) quer zwischen der Innenseite (36) und er Außenseite (26) in Richtung des distalen Endes der Schneidklinge (20) erstreckt und an die Außenseite (26) in einem spitzen Winkel anschließt, um mit der Außenseite (26) die geschärfte Schneidkante (36) zu definieren, wobei die geschärfte Schneidkante (36) in der ersten zylinderförmigen Ebene liegt, in der die Außenseite (26) der Seitenwand (22) im Allgemeinen liegt, **dadurch gekennzeichnet, dass** die Schneidklinge (20) ferner einen Ring (42) aufweist, wobei der Ring (42) auf der Innenseite (30) der Seitenwand (23) angeordnet ist und sich radial von dieser und teilweise in die axiale Bohrung (24) erstreckt, wobei der Ring (42) zum selektiven Eingriff mit dem Gewebegreifinstrument, das während eines chirurgischen Eingriffs zusammen mit dem Morcellator verwendet wird, vorgesehen ist.

4. Schneidklinge (20) für einen chirurgischen Morcellator nach einem der vorhergehenden Ansprüche, worin die geneigte Fläche (34) der Schneidklinge (20) mit der zweiten zylinderförmigen Ebene (32), in der die Innenseite (30) der Schneidklingen-Seitenwand (22) im Allgemeinen liegt, einen Außenwinkel von ca. 15 Grad (15°) bildet.

## Revendications

1. Lame de coupe (20) pour un système de morcellement chirurgical, la lame de coupe (20) présentant une extrémité distale et un chant tranchant de coupe (36) situé sur l'extrémité distale pour sectionner un tissu et former des morceaux de tissu, la lame de coupe (20) étant structurée de façon à minimiser le contact entre le chant tranchant (36) et un instrument de saisie de tissu (8) utilisé avec le système de morcellement au cours d'une opération chirurgicale, la lame de coupe (20) comprenant :
une paroi latérale globalement cylindrique (22) définissant un trou axial pour le passage des morceaux de tissu sectionné, la paroi latérale (22) ayant une surface externe (26) globalement située dans un premier plan cylindrique (28), une surface interne (30) globalement située dans un deuxième plan cylindrique (32) et une surface inclinée (34), la surface interne (30) de la paroi latérale (22) étant disposée radialement à l'intérieur de la surface externe (26) de la paroi latérale (22), la surface inclinée (34) s'étendant transversalement entre la surface interne (30) et la surface externe (26) en direction de l'extrémité distale de la lame de coupe (20) et rejoignant la surface externe (26) à un angle aigu afin de définir le chant tranchant de coupe (36) avec la surface externe (26), le chant tranchant de coupe (36) étant situé dans le premier plan cylindrique (28) sur lequel se trouve globalement la surface externe (26) de la paroi latérale (22),
**caractérisée en ce que**
la surface inclinée (34) de la paroi latérale (22) de la lame de coupe s'étend radialement vers l'intérieur au-delà du deuxième plan cylindrique (32) dans lequel se trouve globalement la surface interne (30) de la paroi latérale (22) de la lame de coupe en définissant une saillie circulaire (38) située radialement vers l'intérieur de la surface interne (30) pour l'engagement sélectif avec l'instrument de saisie de tissu (8) utilisé avec le système de morcellement au cours d'une opération chirurgicale.

2. Lame de coupe (20) pour un système de morcellement chirurgical, la lame de coupe (20) présentant une extrémité distale et un chant tranchant de coupe (36) situé sur l'extrémité distale pour sectionner un tissu et former des morceaux de tissu, la lame de coupe (20) étant structurée de façon à minimiser le contact entre le chant tranchant (36) et un instrument de saisie de tissu (8) utilisé avec le système de morcellement au cours d'une opération chirurgicale, la lame de coupe (20) comprenant :
une paroi latérale globalement cylindrique (22) définissant un trou axial pour le passage des morceaux de tissu sectionné, la paroi latérale (22) ayant une surface externe (26) globalement située dans un premier plan cylindrique (28), une surface interne (30) globalement située dans un deuxième plan cylindrique (32) et une surface inclinée (34), la surface interne (30) de la paroi latérale (22) étant disposée radialement à l'intérieur de la surface externe (26) de la paroi latérale (22), la surface inclinée (34) s'étendant transversalement entre la surface interne (30) et la surface externe (26) en direction de l'extrémité distale de la lame de coupe (20) et rejoignant la surface externe (26) à un angle aigu afin de définir le chant tranchant de coupe (36) avec la surface externe (26), le chant tranchant de coupe (36) étant situé dans le premier plan cylindrique (28) sur lequel se trouve globalement la surface externe (26) de la paroi latérale (22),
**caractérisée en ce que**
la surface interne (30) de la paroi latérale (22) de la lame de coupe comprend une partie s'étendant radialement vers l'extérieur, la surface inclinée (34) de la paroi latérale (22) de la lame de coupe s'étendant entre la partie s'étendant radialement vers l'extérieur et la surface externe (26) de la paroi latérale (22) de la lame de coupe, la partie s'étendant radialement vers l'extérieur de la surface interne (30) définissant un chant radial (40) dans la paroi latérale (22) de la lame de coupe pour l'engagement sélectif avec l'instrument de saisie de tissu (8) utilisé avec le système de morcellement au cours d'une opération chirurgicale.

3. Lame de coupe (20) pour un système de morcellement chirurgical, la lame de coupe (20) présentant une extrémité distale et un chant tranchant de coupe (36) situé sur l'extrémité distale pour sectionner un tissu et former des morceaux de tissu, la lame de coupe (20) étant structurée de façon à minimiser le contact entre le chant tranchant (36) et un instrument de saisie de tissu (8) utilisé avec le système de morcellement au cours d'une opération chirurgicale, la lame de coupe (20) comprenant :
une paroi latérale globalement cylindrique (22) définissant un trou axial pour le passage des morceaux de tissu sectionné, la paroi latérale (22) ayant une surface externe (26) globalement située dans un premier plan cylindrique (28), une surface interne (30) globalement située dans un deuxième plan cylindrique (32) et une surface inclinée (34), la surface interne (30) de la paroi latérale (22) étant disposée radialement à l'intérieur de la surface externe (26) de la paroi latérale (22), la surface inclinée (34) s'étendant transversalement entre la surface interne (30) et la surface externe (26) en direction de l'extrémité distale de la lame de coupe (20) et rejoignant la surface externe (26) à un angle aigu afin de définir le chant tranchant de coupe (36) avec la surface externe (26), le chant tranchant de coupe (36) étant situé dans le premier plan cylindrique (28) sur lequel se trouve globalement la surface externe (26) de la paroi latérale (22),
**caractérisée en ce que**
la lame de coupe (20) comprend en outre un anneau (42), l'anneau (42) étant situé sur la surface interne (30) de la paroi latérale (22) et s'étendant radialement depuis celui-ci et en partie dans le trou axial (24), l'anneau (42) étant prévu pour l'engagement sélectif avec l'instrument de saisie de tissu (8) utilisé avec le système de morcellement au cours d'une opération chirurgicale.

4. Lame de coupe (20) pour un système de morcellement chirurgical selon l'une quelconque des revendications précédentes, dans laquelle la surface inclinée (34) de la lame de coupe (20) forme un angle externe d'environ 15 degrés (15°) avec le deuxième plan cylindrique (32) dans lequel se trouve globalement la surface interne (30) de la paroi latérale (22) de la lame de coupe.
